# EUROPEAN PATENT SPECIFICATION

(11) **EP 3 250 059 B1**
(45) Date of publication and mention of the grant of the patent: **26.06.2019**
(21) Application number: 16701352.3
(22) Date of filing: 22.01.2016
(51) Int. Cl.: A24F 47/00

(54) **AEROSOL-GENERATING ARTICLE WITH INTEGRAL HEATING ELEMENT**
AEROSOLERZEUGENDER ARTIKEL MIT INTEGRIERTEM HEIZELEMENT
ARTICLE DE GÉNÉRATION D'AÉROSOL AVEC ÉLÉMENT CHAUFFANT INTÉGRÉ

(30) Priority: 28.01.2015 EP 15152940
(43) Date of publication of application: 06.12.2017
(73) Proprietor: Philip Morris Products S.A., 2000 Neuchâtel (CH)
(72) Inventor: RUSCIO, Dani, 2088 Cressier (CH); BATISTA, Rui Nuno, 1110 Morges (CH); MIRONOV, Oleg, 2000 Neuchatel (CH); PLOJOUX, Julien, 1208 Geneva (CH)
(74) Representative: Dowling, Ian
(86) International application number: PCT/EP2016/051385
(87) International publication number: WO 2016/120177

(56) References cited:
- EP-A1- 2 022 349
- WO-A1-2013/034456
- WO-A1-2013/178769

## Description

The present invention relates to an aerosol-generating article having an integrated heating element. The present invention also relates to an electrically heated aerosol-generating system comprising an aerosol-generating article having an integrated heating element, and to methods of manufacturing tobacco plugs and aerosol-generating articles each having an integrated heating element.

One type of aerosol-generating system is an electrically operated smoking system.

EP-2-022-349-A1 describes a known electrically operated smoking system comprising an atomiser for vaporising a nicotine-containing liquid.

WO 2013/034456 A1 describes an apparatus for heating a smokable material, the apparatus comprising a heater and a heating chamber extending around the heater. A cartridge comprising a smokable material may be inserted into the heating chamber so that the heater is received within the cartridge. The smokable material may comprise tobacco. The apparatus also comprises a mouthpiece.

Other known handheld electrically operated smoking systems typically comprise an aerosol-generating device comprising a battery, control electronics and an electric heater for heating a smoking article designed specifically for use with the aerosol-generating device. In some examples, the smoking article comprises a plug of an aerosol-forming substrate, such as a tobacco plug, and the heater contained within the aerosol-generating device is inserted into the aerosol-forming substrate when the smoking article is inserted into the aerosol-generating device. However, the electric heater may become contaminated with material from the aerosol-forming substrate during use and cleaning the electric heater inside the device can be difficult. In some cases, it may be necessary to dispose of the entire device if the heater cannot be adequately cleaned. Sometimes removal of the smoking article from the device is also difficult, which may result in the need for a dedicated extraction tool to facilitate removal of the smoking article from the device without damaging the heater.

Accordingly, it would be desirable to produce an aerosol-generating article and an electrically heated aerosol-generating system that address the issue of heater contamination and the difficulty in extracting the article from an aerosol-generating device.

According to a first aspect of the present invention there is provided an aerosol-generating article comprising a tobacco plug, a mouthpiece positioned downstream of the tobacco plug, and a resistive heating element positioned within the tobacco plug. The resistive heating element comprises an upstream portion protruding from an upstream end of the tobacco plug, wherein the upstream portion of the resistive heating element comprises at least two heater electrical contacts for receiving a supply of electrical energy from at least two device electrical contacts when the aerosol-generating article comprising the resistive heating element is inserted into an aerosol-generating device.

As used herein, the term "aerosol-generating article" refers to an article comprising a tobacco plug that, when heated, releases volatile compounds that can form an aerosol.

As used herein, the term "aerosol-generating device" refers to a device that interacts with an aerosol-generating article to generate an aerosol. The aerosol-generative device includes a supply of electrical energy to operate the resistive heating element within the aerosol-generating article.

As used herein, the terms "upstream" and "downstream" are used to describe the relative positions of components, or portions of components, of aerosol-generating articles according to the invention with respect to the direction of airflow through the aerosol-generating article when a user draws on the aerosol-generating article. In particular, when a user draws on the article, air flows in the downstream direction from the upstream end to the downstream end.

By providing a resistive heating element as part of the aerosol-generating article, rather than part of an aerosol-generating device, the present invention addresses at least some of the issues associated with known electrically heated aerosol-generating systems. In particular, providing the aerosol-generating article with an integrated resistive heating element eliminates the need to remove contamination from a heating element contained within an aerosol-generating device, as is the case with known electrically heated aerosol-generating systems in which the heater is a reusable heater fixed within an aerosol-generating device. Providing the resistive heating element in the aerosol-generating article can also make it easier to remove the article from an aerosol-generating device when compared to known electrically heated aerosol-generating systems in which a fixed heating element within the device can make it difficult to remove the article without the use of a dedicated extraction tool.

In some embodiments, the resistive heating element may comprise an electrically resistive heating track provided on an elongate electrically insulating substrate, and wherein the electrically resistive heating track is electrically connected to the at least two heater electrical contacts. Providing an electrically resistive heating track on an electrically insulating substrate can simplify the manufacture of the aerosol-generating article by facilitating separate manufacture of the resistive heating element, which can then be inserted into the tobacco plug after the remainder of the aerosol-generating article has been constructed.

The resistive heating element may be provided to a consumer separately from the remainder of the aerosol-generating article so that the consumer can insert the resistive heating element into the tobacco plug. This arrangement is particularly useful in embodiments in which the resistive heating element is reusable with a plurality of aerosol-generating articles. Alternatively, a resistive heating element may be inserted into each tobacco plug during the manufacture of the aerosol-generating articles.

To facilitate the insertion of the resistive heating element into the tobacco plug, the elongate electrically insulating substrate is preferably shaped in the form of one of a rod, a needle, a pin, a blade, or a cone. Additionally, or alternatively, the elongate electrically insulating substrate may comprise one or more serrated edges, wherein the serrations are shaped to favour insertion of the resistive heating element into the tobacco plug and resist removal of the resistive heating element from the tobacco plug. Providing such serrated edges can advantageously prevent the resistive heating element from becoming dislodged from the tobacco plug when the aerosol-generating article is removed from an aerosol-generating device.

In alternative embodiments, the elongate electrically insulating substrate may have a helical shape so that the resistive heating element can be screwed into and out of the tobacco plug but cannot be pushed directly into or pulled directly out of the tobacco plug. Utilising a helical shape can therefore prevent the resistive heating element from becoming dislodged from the tobacco plug when the aerosol-generating article is removed from an aerosol-generating device.

In those embodiments in which a resistive heating element is inserted into each tobacco plug during the manufacture of aerosol-generating articles according to the present invention, the resistive heating element is typically disposable with the remainder of the aerosol-generating article. In such embodiments, the resistive heating element is preferably constructed from low-cost materials, such as low cost metals and metal alloys.

Alternatively, the resistive heating element may be reusable with multiple aerosol-generating articles. For example, a pack of aerosol-generating articles may be provided with a single resistive heating element, wherein the resistive heating element is removed from the tobacco plug of each aerosol-generating article after it has been smoked and inserted into the next aerosol-generating article. In such embodiments, it may be cost-effective to manufacture the resistive heating element from more expensive materials. For example, reusable resistive heating elements may comprise an elongate electrically insulating substrate formed from a non-conductive ceramic.

Preferably, the electrically insulating substrate is operable at a working temperature of up to about 700 degrees Celsius, more preferably about 800 degrees Celsius. Additionally, or alternatively, the operating temperature of the resistive heating element during use may be about 250 degrees Celsius, more preferably about 300 degrees Celsius.

Suitable materials for forming the electrically resistive heating track include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal® and iron-manganese-aluminium based alloys.

In some embodiments, the electrically resistive heating track comprises one or more stamped portions of electrically resistive material, such as stainless steel. Alternatively, the electrically resistive heating track may comprise a heating wire or filament, for example a Ni-Cr (Nickel-Chromium), platinum, tungsten or alloy wire.

In any of those embodiments in which the resistive heating element comprises an electrically resistive heating track provided on an elongate electrically insulating substrate, the upstream portion of the resistive heating element may comprises a disc of electrically insulating material, wherein the disc comprises a downstream face abutting an upstream end of the tobacco plug, wherein the elongate electrically insulating substrate extends downstream from the downstream face of the disc, and wherein the disc further comprises an upstream face on which the at least two heater electrical contacts are provided.

Providing the resistive heating element with a disc of electrically insulating material at its upstream end advantageously facilitates insertion of the resistive heating element into the tobacco plug in those embodiments in which the resistive heating element is inserted into the tobacco plug after the tobacco plug has been formed. Specifically, the elongate electrically insulating substrate can be sized so that when the resistive heating element is inserted into the tobacco plug so that the downstream face of the disc of electrically insulating material abuts the upstream end of the tobacco plug the elongate electrically insulating substrate, and therefore the resistive heating track, are correctly positioned within the tobacco plug.

Advantageously, the upstream face of the disc of electrically insulating material also provides a large surface area on which the at least two heater electrical contacts can be provided, which facilitates the manufacture of the resistive heating element.

The disc of electrically insulating material may be formed from the same insulating material as the elongate electrically insulating substrate, or they may be formed from different materials. Preferably, the disc of electrically insulating material and the elongate electrically insulating substrate are formed from the same material. To simplify the manufacture of the resistive heating element, the disc of electrically insulating material and the elongate electrically insulating substrate are preferably integrally formed from a single piece of electrically insulating material. For example, the resistive heating element may formed by moulding or otherwise casting an electrically insulating material around the electrically resistive heating track.

In any of those embodiments in which the resistive heating element comprises a disc of electrically insulating material, the disc and the tobacco plug preferably both have a substantially circular cross-sectional shape, wherein the diameter of the disc is substantially the same as the diameter of the tobacco plug. This advantageously facilitates correct insertion of the resistive heating element into the tobacco plug. For example, in those embodiments in which a resistive heating element is inserted into each tobacco plug during the manufacture of the aerosol-generating articles, a tubular guide may be used to ensure that the disc of electrically insulating material is positioned coaxially with the tobacco plug.

In any of the embodiments described above, the aerosol-generating article preferably further comprises an article securing element configured to interact with a device securing element on an aerosol-generating device to releasably retain the aerosol-generating article within the aerosol-generating device. By releasably retaining the aerosol-generating article within the aerosol-generating device the article securing element can advantageously ensure that a reliable electrical contact is maintained between the heater electrical contacts and corresponding device electrical contacts in the aerosol-generating device during heating of the aerosol-generating article.

The article securing element is preferably configured so that forces exerted on the aerosol-generating article during normal use of an electrically heated aerosol-generating system comprising the aerosol-generating article inserted into an aerosol-generating device are insufficient to break the electrical contact between the heater electrical contacts and device electrical contacts, while also being configured so that a consumer can easily remove the aerosol-generating article, including the resistive heating element, from the aerosol-generating device. For example, the resistive heater element may comprise a magnetised material provided at an upstream end of the resistive heater element, wherein the magnetised material interacts with a magnetised material or a non-magnetised but ferromagnetic material provided within the aerosol-generating device. In a particularly preferred embodiment, at least one of the heater electrical contacts comprises a magnetised material, wherein the at least one heater electrical contact comprising a magnetised material is magnetically attracted to the corresponding at least one device electrical contact to releasably retain the heater electrical contacts in electrical contact with the corresponding device electrical contacts when the aerosol-generating article is inserted into the aerosol-generating device. In such embodiments, the article securing element comprises the at least one heater electrical contact comprising a magnetised material. The one or more device electrical contacts corresponding to the at least one heater electrical contact comprising a magnetised material may also comprise a magnetised material. Alternatively, the one or more device electrical contacts corresponding to the at least one heater electrical contact comprising a magnetised material may comprise a non-magnetised but ferromagnetic material.

In an alternative embodiment, at least one of the device electrical contacts comprises a magnetised material, wherein the at least one device electrical contact comprising a magnetised material is magnetically attracted to the corresponding at least one heater electrical contact to releasably retain the heater electrical contacts in electrical contact with the corresponding device electrical contacts when the aerosol-generating article is inserted into the aerosol-generating device. In such embodiments, the device securing element comprises the at least one device electrical contact comprising a magnetised material and the article securing element comprises the one or more corresponding heater electrical contacts. The one or more heater electrical contacts corresponding to the at least one device electrical contact comprising a magnetised material may also comprise a magnetised material. Alternatively, the one or more heater electrical contacts corresponding to the at least one device electrical contact comprising a magnetised material may comprise a non-magnetised but ferromagnetic material.

In further alternative embodiments, the article and device securing elements may interact mechanically to retain the aerosol-generating article within the aerosol-generating device. For example, the aerosol-generating device may comprise a tapered cavity into which the aerosol-generating article is received, the tapered cavity providing an interference fit between the tapered surface of the cavity and the outer surface of the aerosol-generating article. In such embodiments, the cavity forms the device securing element and the outer surface of the aerosol-generating article forms the article securing element. Alternatively, a different mechanical interaction may be provided between the aerosol-generating article and the aerosol-generating device. For example, the aerosol-generating article may comprise a male portion of a bayonet connection that interacts with a corresponding female portion of a bayonet connection provided within the aerosol-generating device. In such embodiments, the male and female portions of the bayonet connection form the article and device securing elements respectively. In those embodiments in which the resistive heating element comprises a disc of electrically insulating material the male portion of the bayonet connection is preferably provided by the disc of electrically insulating material.

In any of the embodiments described above, the aerosol-generating article may have a substantially circular cross-sectional shape. In such embodiments, the at least two heater electrical contacts may comprise a first heater electrical contact spaced apart from a second heater electrical contact, wherein the aerosol-generating article further comprises an indexing indicator to indicate the rotational orientation of the aerosol-generating article. Providing an indexing indicator on the aerosol-generating article advantageously assists the consumer in inserting the aerosol-generating article into an aerosol-generating device in the correct orientation so that the at least two heater electrical contacts are aligned with and contact corresponding device electrical contacts as may be provided inside the aerosol-generating device.

For example, the aerosol-generating article may comprise a shaped portion, such as a raised portion or an indentation, which forms the indexing indicator. In such embodiments, the indexing indicator may cooperate with a correspondingly shaped portion on an aerosol-generating device. For example, the aerosol-generating article may comprise a groove in an outer surface of the article which must be aligned with a correspondingly shaped ridge on an interior surface of an aerosol-generating device to allow insertion of the article into the device.

Additionally, or alternatively, the indexing indicator may comprise one or more indicia provided on an outer surface of the aerosol-generating article, which must be aligned with a corresponding indicia provided on an aerosol-generating device. In such embodiments, at least one indicia is preferably provided at the downstream end of the aerosol-generating article so that it remains visible to the consumer when the aerosol-generating article has been inserted into an aerosol-generating device.

In alternative embodiments, the aerosol-generating article may have a substantially circular cross-sectional shape centred on a longitudinal axis of the aerosol-generating article, wherein the at least two heater electrical contacts comprise a first heater electrical contact having an annular shape centred on the longitudinal axis of the aerosol-generating article and a second heater electrical contact having a circular or an annular shape centred on the longitudinal axis of the aerosol-generating article. An outer diameter of the second heater electrical contact is smaller than an inner diameter of the first heater electrical contact, and the second heater electrical contact is positioned within the first heater electrical contact.

Advantageously, forming a first of the heater electrical contacts as an annular contact with the second heater contact coaxially positioned within the first heater electrical contact and on the longitudinal axis of the aerosol-generating article provides a heater electrical contact configuration that is independent of the rotational orientation of the aerosol-generating article. Therefore, in such embodiments, an indexing indicator may not be required as the correct contact between the heater electrical contacts and device electrical contacts in an aerosol-generating device may be achieved with any rotational orientation of the article with respect to the device.

In any of the embodiments described above, the aerosol-generating article may further comprise an article data storage device for storing data indicative of the type of aerosol-generating article. Additionally, or alternatively, the article data storage device may store data relating to a heating profile for the aerosol-generating article.

In those embodiments in which the aerosol-generating article comprises an article data storage device, the aerosol-generating article preferably further comprises one or more article data electrical contacts for connecting to one or more device data electrical contacts on an aerosol-generating device.

At least one of the article data storage device and the article data electrical contacts may be configured so that the article data storage device can be accessed only in a read-only mode. For example, the article data storage device may communicate the type of aerosol-generating article to an aerosol-generating device via the article data electrical contacts and device data electrical contacts so that the aerosol-generating device can select the appropriate heating profile from one or more heating profiles stored within a device data storage device. Alternatively, the article data storage device may communicate an appropriate heating profile to an aerosol-generating device via the article data electrical contacts and device data electrical contacts.

Alternatively, at least one of the article data storage device and the article data electrical contacts may be configured so that the article data storage device can be accessed in a read-write mode. For example, an aerosol-generating device may read at least one of the type of aerosol-generating article and an appropriate heating profile from the article data storage device, as described above. Additionally, or alternatively, an aerosol-generating device may write data to the article data storage device. For example, an aerosol-generating device may write data to the article data storage device to indicate that the aerosol-generating article has been smoked. If the smoked aerosol-generating article is then re-inserted into an aerosol-generating device, the aerosol-generating device may read the data from the article data storage device indicative of the aerosol-generating article having been smoked and therefore prevent the article being smoked again.

In those embodiments in which the aerosol-generating article comprises an article data storage device, the article data storage device is preferably provided on or otherwise formed integrally with the resistive heating element. For example, in those embodiments in which the resistive heating element comprises a disc of electrically insulting material, the article data storage device is preferably provided on or within the disc of electrically insulating material. Similarly, in those embodiments in which the aerosol-generating article comprises one or more article data electrical contacts, the article data electrical contacts are preferably provided on the upstream face of the disc of electrically insulating material.

In any of the embodiments described above, the tobacco plug may comprise at least one an aerosol former, that is, a substance which generates an aerosol upon heating. The aerosol former may be, for instance, a polyol aerosol former or a non-polyol aerosol former. It may be a solid or liquid at room temperature. Suitable polyols include sorbitol, glycerol, and glycols like propylene glycol or triethylene glycol. Suitable non-polyols include monohydric alcohols, such as menthol, high boiling point hydrocarbons, acids such as lactic acid, and esters such as diacetin, triacetin, triethyl citrate or isopropyl myristate. Aliphatic carboxylic acid esters such as methyl stearate, dimethyl dodecanedioate and dimethyl tetradecanedioate can also be used as aerosol formers. A combination of aerosol formers may be used, in equal or differing proportions. Polyethylene glycol and glycerol may be particularly preferred, whilst triacetin is more difficult to stabilise and may also need to be encapsulated in order to prevent its migration within the article. The at least one aerosol-forming substrate may include one or more flavouring agents, such as cocoa, liquorice, organic acids, or menthol.

The tobacco plug may comprise one or more of: powder, granules, pellets, shreds, spaghettis, strips or sheets containing one or more of: tobacco leaf, fragments of tobacco ribs, reconstituted tobacco, homogenised tobacco, extruded tobacco and expanded tobacco. Optionally, the tobacco plug may contain additional tobacco or non-tobacco volatile flavour compounds, to be released upon heating of the tobacco plug. Optionally, the tobacco plug may also contain capsules that, for example, include the additional tobacco or non-tobacco volatile flavour compounds. Such capsules may melt during heating of the tobacco plug. Alternatively, or in addition, such capsules may be crushed prior to, during, or after heating of the tobacco plug.

Where the tobacco plug comprises homogenised tobacco material, the homogenised tobacco material may be formed by agglomerating particulate tobacco. The homogenised tobacco material may be in the form of a sheet. The homogenised tobacco material may have an aerosol-former content of greater than 5 percent on a dry weight basis. The homogenised tobacco material may alternatively have an aerosol former content of between 5 percent and 30 percent by weight on a dry weight basis. Sheets of homogenised tobacco material may be formed by agglomerating particulate tobacco obtained by grinding or otherwise comminuting one or both of tobacco leaf lamina and tobacco leaf stems; alternatively, or in addition, sheets of homogenised tobacco material may comprise one or more of tobacco dust, tobacco fines and other particulate tobacco by-products formed during, for example, the treating, handling and shipping of tobacco. Sheets of homogenised tobacco material may comprise one or more intrinsic binders, that is tobacco endogenous binders, one or more extrinsic binders, that is tobacco exogenous binders, or a combination thereof to help agglomerate the particulate tobacco. Alternatively, or in addition, sheets of homogenised tobacco material may comprise other additives including, but not limited to, tobacco and non-tobacco fibres, aerosol-formers, humectants, plasticisers, flavourants, fillers, aqueous and non-aqueous solvents and combinations thereof. Sheets of homogenised tobacco material are preferably formed by a casting process of the type generally comprising casting a slurry comprising particulate tobacco and one or more binders onto a conveyor belt or other support surface, drying the cast slurry to form a sheet of homogenised tobacco material and removing the sheet of homogenised tobacco material from the support surface.

The aerosol-generating article may have a total length of between approximately 30 millimetres and 100 millimetres. The aerosol-generating article may have an external diameter of between approximately 5 millimetres and approximately 13 millimetres.

The mouthpiece may be located at the downstream end of the aerosol-generating article. The mouthpiece may be a cellulose acetate filter plug. The mouthpiece is preferably approximately 7 millimetres in length, but can have a length of between approximately 5 millimetres to approximately 10 millimetres.

The tobacco plug may have a length of approximately 10 millimetres. However it is most preferable for the tobacco plug to have a length of 12 millimetres.

The diameter of the tobacco plug may be between approximately 5 millimetres and approximately 12 millimetres.

Preferably, the aerosol-generating article is a cigarette. In a preferred embodiment, the aerosol-generating article has a total length between 40 millimetres and 50 millimetres. Preferably, the aerosol-generating article has a total length of approximately 45 millimetres. It is also preferable for the aerosol-generating article to have an external diameter of approximately 7.2 millimetres.

The present invention also extends to electrically heated aerosol-generating systems comprising the aerosol-generating article described above. Therefore, according to a second aspect of the present invention there is provided an electrically heated aerosol-generating system comprising an aerosol-generating article according to the first aspect of the present invention, in accordance with any of the embodiments described above, and an aerosol-generating device. The aerosol-generating device comprises a tubular housing comprising a cavity for receiving at least an upstream portion of the aerosol-generating article. The aerosol-generating device also comprises a supply of electrical energy within the tubular housing, and at least two device electrical contacts connected to the supply of electrical energy. The at least two device electrical contacts are positioned at an upstream end of the cavity and configured to contact the at least two heater electrical contacts when the aerosol-generating article is inserted into the cavity to transfer electrical energy from the supply of electrical energy to the resistive heating element.

In some embodiments, the aerosol-generating article and the cavity may both have a substantially circular cross-sectional shape. In such embodiments, the at least two heater electrical contacts may comprise a first heater electrical contact spaced apart from a second heater electrical contact, and the at least two device electrical contacts may comprise a first device electrical contact spaced apart from a second device electrical contact. The aerosol-generating article and the aerosol-generating device may each comprise an indexing indicator to indicate the rotational orientation of the aerosol-generating article with respect to the aerosol-generating device when the aerosol-generating article is received within the cavity. As described above, providing an indexing indicator on each of the aerosol-generating article and the aerosol-generating device advantageously assists the consumer in inserting the aerosol-generating article into the aerosol-generating device in the correct orientation so that the at least two heater electrical contacts are aligned with and contact the corresponding device electrical contacts.

In any of the embodiments described above in which the aerosol-generating article comprises an article securing element, the aerosol-generating device preferably comprises a device securing element configure to interact with the article securing element to releasably retain the aerosol-generating article within the aerosol-generating device. By releasably retaining the aerosol-generating article within the aerosol-generating device the article securing element and the device securing element can advantageously ensure that a reliable electrical contact is maintained between the heater electrical contacts and the corresponding device electrical contacts during heating of the aerosol-generating article.

The article securing element and the device securing element are preferably configured so that forces exerted on the aerosol-generating article during normal use of the electrically heated aerosol-generating system are insufficient to break the electrical contact between the heater electrical contacts and the device electrical contacts, while also being configured so that a consumer can easily remove the aerosol-generating article, including the resistive heating element, from the aerosol-generating device. For example, the resistive heater element may comprise a magnetised material provided at an upstream end of the resistive heater element, wherein the magnetised material interacts with a magnetised material or a non-magnetised but ferromagnetic material provided within the aerosol-generating device. Alternatively, the aerosol-generating device may comprise a magnetised material configured to interact with a magnetised material or a non-magnetised but ferromagnetic material provided at an upstream end of the resistive heater element.

In a preferred embodiment, at least one of the heater electrical contacts comprises a magnetised material, wherein the at least one heater electrical contact comprising a magnetised material is magnetically attracted to the corresponding at least one device electrical contact to releasably retain the heater electrical contacts in electrical contact with the corresponding device electrical contacts when the aerosol-generating article is inserted into the aerosol-generating device. In such embodiments, the article securing element comprises the at least one heater electrical contact comprising a magnetised material and the device securing element comprises the one or more corresponding device electrical contacts. The one or more device electrical contacts corresponding to the at least one heater electrical contact comprising a magnetised material may also comprise a magnetised material. Alternatively, the one or more device electrical contacts corresponding to the at least one heater electrical contact comprising a magnetised material may comprise a non-magnetised but ferromagnetic material.

In an alternative embodiment, at least one of the device electrical contacts comprises a magnetised material, wherein the at least one device electrical contact comprising a magnetised material is magnetically attracted to the corresponding at least one heater electrical contact to releasably retain the heater electrical contacts in electrical contact with the corresponding device electrical contacts when the aerosol-generating article is inserted into the aerosol-generating device. In such embodiments, the device securing element comprises the at least one device electrical contact comprising a magnetised material and the article securing element comprises the one or more corresponding heater electrical contacts. The one or more heater electrical contacts corresponding to the at least one device electrical contact comprising a magnetised material may also comprise a magnetised material. Alternatively, the one or more heater electrical contacts corresponding to the at least one device electrical contact comprising a magnetised material may comprise a non-magnetised but ferromagnetic material.

In further alternative embodiments, the article and device securing elements may interact mechanically to retain the aerosol-generating article within the aerosol-generating device. For example, the cavity into which the aerosol-generating article is received may be tapered, the tapered cavity providing an interference fit between the tapered surface of the cavity and the outer surface of the aerosol-generating article. In such embodiments, the cavity forms the device securing element and the outer surface of the aerosol-generating article forms the article securing element. Alternatively, a different mechanical interaction may be provided between the aerosol-generating article and the aerosol-generating device. For example, the aerosol-generating article may comprise a male portion of a bayonet connection that interacts with a corresponding female portion of a bayonet connection provided within the aerosol-generating device. In such embodiments, the male and female portions of the bayonet connection form the article and device securing elements respectively. In those embodiments in which the resistive heating element comprises a disc of electrically insulating material the male portion of the bayonet connection is preferably provided by the disc of electrically insulating material.

For example, the aerosol-generating article may comprise a shaped portion, such as a raised portion or an indentation, which forms an indexing indicator on the aerosol-generating article that cooperates with a correspondingly shaped indexing indicator on the aerosol-generating device. For example, the aerosol-generating article may comprise a groove in an outer surface of the article which must be aligned with a correspondingly shaped ridge on the interior surface of the cavity to allow insertion of the article into the cavity

Alternatively, the at least two device electrical contacts may comprise a first device electrical contact spaced apart from a second device electrical contact, wherein the aerosol-generating article has a substantially circular cross-sectional shape centred on a longitudinal axis of the aerosol-generating article, and wherein the at least two heater electrical contacts comprise a first heater electrical contact having an annular shape centred on the longitudinal axis of the aerosol-generating article and a second heater electrical contact having a circular or an annular shape centred on the longitudinal axis of the aerosol-generating article. The outer diameter of the second heater electrical contact is smaller than an inner diameter of the first heater electrical contact, and the second heater electrical contact is positioned within the first heater electrical contact.

As described above, forming a first of the heater electrical contacts as an annular contact with the second heater contact coaxially positioned within the first heater electrical contact and on the longitudinal axis of the aerosol-generating article advantageously provides a heater electrical contact configuration that is independent of the rotational orientation of the aerosol-generating article. Therefore, in such embodiments, an indexing indicator may not be required as the correct contact between the heater electrical contacts and the device electrical contacts in the aerosol-generating device may be achieved with any rotational orientation of the article with respect to the device, providing one of the device electrical contacts is centred on a longitudinal axis of the cavity and the spacing between the first and second device electrical contacts is the same as the spacing between the first and second heater electrical contacts. In some embodiments, the first and second device electrical contacts may have the same configuration as the heater electrical contacts. That is, the first device electrical contact may be an annular electrical contact surrounding the second device electrical contact.

In alternative embodiments, the first device electrical contact may be an annular electrical contact surrounding the second device electrical contact, both centred on the longitudinal axis of the cavity, and the first and second heater electrical contacts may have any configuration, providing one of the heater electrical contacts is centred on the longitudinal axis of the aerosol-generating article and the spacing between the first and second heater electrical contacts is the same as the spacing between the first and second device electrical contacts. Such a configuration still permits insertion of the aerosol-generating article into the aerosol-generating device with any rotational orientation.

In any of the embodiments described above in which the aerosol-generating article comprises an article data storage device and one or more article data electrical contacts, the aerosol-generating device preferably comprises one or more device data electrical contacts for connecting to the one or more article data electrical contacts.

At least one of the article data storage device and the article data electrical contacts may be configured so that the article data storage device can be accessed only in a read-only mode. For example, the aerosol-generating device may comprise a device data storage device storing one or more heating profiles, wherein the article data storage device communicates the type of aerosol-generating article to the aerosol-generating device via the article data electrical contacts and the device data electrical contacts so that the aerosol-generating device can select the appropriate heating profile from the one or more heating profiles stored within the device data storage device. Alternatively, the article data storage device may communicate an appropriate heating profile to the aerosol-generating device via the article data electrical contacts and the device data electrical contacts.

Alternatively, at least one of the article data storage device and the article data electrical contacts may be configured so that the article data storage device can be accessed in a read-write mode. For example, the aerosol-generating device may read at least one of the type of aerosol-generating article and an appropriate heating profile from the article data storage device, as described above. Additionally, or alternatively, the aerosol-generating device may write data to the article data storage device. For example, the aerosol-generating device may write data to the article data storage device to indicate that the aerosol-generating article has been smoked. If the smoked aerosol-generating article is then re-inserted into an aerosol-generating device, the aerosol-generating device may read the data from the article data storage device indicative of the aerosol-generating article having been smoked and therefore prevent the article being smoked again.

In those embodiments in which the aerosol-generating device comprises a device data storage device, the device data storage device may store one or more heating profiles, as described above. Additionally, or alternatively, the device data storage device may store usage data, such as the number of article smokes, the types of articles smoked, the frequency of smoking, and such like.

The supply of electrical energy may be a DC voltage source. In preferred embodiments, the supply of electrical energy is a battery. For example, the supply of electrical energy may be a nickel-metal hydride battery, a nickel cadmium battery, or a lithium based battery, for example a lithium-cobalt, a lithium-iron-phosphate or a lithium-polymer battery. The supply of electrical energy may alternatively be another form of charge storage device such as a capacitor. The supply of electrical energy may require recharging and may have a capacity that allows for the storage of enough energy for use of the aerosol-generating device with one or more aerosol-generating articles.

In any of the embodiments described above, the tubular housing preferably comprises one or more airflow inlets to allow air to flow into the electrically heated aerosol-generating system when a consumer draws on the aerosol-generating article during use. Preferably, the one or more airflow inlets are positioned adjacent the upstream end of the aerosol-generating article when the aerosol-generating article is fully inserted into the aerosol-generating device.

The aerosol-generating device may further comprise a sensor to detect air flow indicative of a consumer taking a puff. The air flow sensor may be an electro-mechanical device. Alternatively, the air flow sensor may be any of: a mechanical device, an optical device, an opto-mechanical device and a micro electro-mechanical systems (MEMS) based sensor. Alternatively, the aerosol-generating device may comprise a manually operable switch for a consumer to initiate a puff.

Additionally, or alternatively, the aerosol-generating device may further comprise a temperature sensor. The temperature sensor may detect the temperature of the resistive heating element or the temperature of the aerosol-generating article. The temperature sensor may be a thermistor. Alternatively, the temperature sensor may comprise a circuit configured to measure the resistivity of the resistive heating element and derive a temperature of the resistive heating element by comparing the measured resistivity to a calibrated curve of resistivity against temperature.

Preferably, the aerosol-generating device comprises an indicator for indicating when the resistive heating element is activated. The indicator may comprise a light, activated when the heating element is activated. Additionally, or alternatively, the aerosol-generating device may comprise an indicator for indicating when the heater electrical contacts are in correct contact with the device electrical contacts. In those embodiments in which the aerosol-generating device comprises indicators for indicating when the resistive heating element is activated and when the heater electrical contacts are in correct contact with the device electrical contacts the indicators may be different indicators or they may be separate indicators. For example, the device may comprise a single indicator comprising a light that activates only when the heater electrical contacts are in correct contact with the device electrical contacts and when the resistive heating element is activated.

In any of the embodiments described above, the aerosol-generating device may comprise an external plug or socket allowing the aerosol-generating device to be connected to another electrical device. For example, the aerosol-generating device may comprise a USB plug or a USB socket to allow connection of the aerosol-generating device to another USB enabled device. For example, the USB plug or socket may allow connection of the aerosol-generating device to a USB charging device to charge a rechargeable supply of electrical energy within the aerosol-generating device. In those embodiments in which the aerosol-generating device comprises a device data storage device, the USB plug or socket may additionally, or alternatively, support the transfer of data to or from, or both to and from, the aerosol-generating device. For example, the device may be connected to a computer to download data from the device data storage device, such as usage data. Additionally, or alternatively, the device may be connected to a computer to transfer data to the device, such as new heating profiles for new aerosol-generating articles, wherein the heating profiles are stored within the device data storage device.

In those embodiments in which the device comprises a USB plug or socket, the device may further comprise a removable cover that covers the USB plug or socket when not in use. In embodiments in which the USB plug or socket is a USB plug, the USB plug may additionally or alternatively be selectively retractable within the device.

As described above, the resistive heating element may comprise an electrically resistive heating track provided on an elongate electrically insulating substrate. The present invention extends to methods of manufacturing aerosol-generating articles comprising such a resistive heating element, in accordance with any of the embodiments described above. Therefore, according to a third aspect of the present invention there is provided a method of manufacturing an aerosol-generating article according to the first aspect of the present invention in accordance with the embodiments described above, the method comprising providing a tobacco plug and a mouthpiece, and wrapping a wrapper around at least a portion of the tobacco plug and the mouthpiece to secure the mouthpiece to a downstream end of the tobacco plug. A resistive heating element is provided, the resistive heating element comprising an electrically resistive heating track on an elongate electrically insulating substrate, and at least two heater electrical contacts for receiving a supply of electrical energy and connected to the electrically resistive heating track. At least a portion of the elongate electrically insulating substrate is inserted into the tobacco plug at an upstream end of the tobacco plug, wherein an upstream portion of the resistive heating element protrudes from the upstream end of the tobacco plug, and wherein the upstream portion of the resistive heating element comprises the at least two heater electrical contacts for receiving a supply of electrical energy.

Providing an electrically resistive heating track on an electrically insulating substrate simplifies the manufacture of the aerosol-generating article by facilitating separate manufacture of the resistive heating element, which is then inserted into the tobacco plug after the remainder of the aerosol-generating article has been constructed.

To facilitate the insertion of the resistive heating element into the tobacco plug, the elongate electrically insulating substrate is preferably shaped in the form of one of a rod, a needle, a pin, a blade, or a cone, and wherein the step of inserting at least a portion of the elongate electrically insulating substrate into the tobacco plug comprises inserting the rod, needle, pin, blade or cone into the tobacco rod.

Additionally, or alternatively, the elongate electrically insulating substrate may comprise one or more serrated edges, wherein the serrations are shaped to favour insertion of the resistive heating element into the tobacco plug during the step of inserting at least a portion of the elongate electrically insulating substrate into the tobacco plug, and wherein the serrations are shaped to resist removal of the resistive heating element from the tobacco plug. Providing such serrated edges can advantageously prevent the resistive heating element from becoming dislodged from the tobacco plug when the aerosol-generating article is removed from an aerosol-generating device.

In alternative embodiments, the elongate electrically insulating substrate may have a helical shape so that the resistive heating element can be screwed into and out of the tobacco plug but cannot be pushed directly into or pulled directly out of the tobacco plug. In such embodiments, the step of inserting at least a portion of the elongate electrically insulating substrate into the tobacco plug comprises screwing at least a portion of the elongate electrically insulating substrate into the tobacco plug. Utilising a helical shape can prevent the resistive heating element from becoming dislodged from the tobacco plug when the aerosol-generating article is removed from an aerosol-generating device.

In those embodiments in which the resistive heating element is disposable with the remainder of the aerosol-generating article, the resistive heating element is preferably constructed from low-cost materials, such as low cost metals and metal alloys. Alternatively, in those embodiments in which the resistive heating element is reusable, it may be cost-effective to manufacture the resistive heating element from more expensive materials. For example, reusable resistive heating elements may comprise an elongate electrically insulating substrate formed from a non-conductive ceramic.

Preferably, the electrically insulating substrate is operable at a working temperature of up to about 700 degrees Celsius, more preferably about 800 degrees Celsius. Additionally, or alternatively, the operating temperature of the resistive heating element during use may be about 250 degrees Celsius, more preferably about 300 degrees Celsius.

Suitable materials for forming the electrically resistive heating track include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal® and iron-manganese-aluminium based alloys.

In some embodiments, the electrically resistive heating track comprises one or more stamped portions of electrically resistive material, such as stainless steel. Alternatively, the electrically resistive heating track may comprise a heating wire or filament, for example a Ni-Cr (Nickel-Chromium), platinum, tungsten or alloy wire.

In any of the embodiments described above, the upstream portion of the resistive heating element preferably comprises a disc of electrically insulating material, wherein the disc comprises an upstream face on which the at least two heater electrical contacts are provided and a downstream face from which the electrically insulating substrate extends. The step of inserting at least a portion of the elongate electrically insulating substrate into the tobacco plug comprises inserting the elongate electrically insulating substrate until the downstream face of the disc abuts the upstream end of the tobacco plug.

Providing the resistive heating element with a disc of electrically insulating material at its upstream end advantageously facilitates insertion of the resistive heating element into the tobacco plug. Specifically, the elongate electrically insulating substrate can be sized so that when the resistive heating element is inserted into the tobacco plug so that the downstream face of the disc of electrically insulating material abuts the upstream end of the tobacco plug the elongate electrically insulating substrate, and therefore the resistive heating track, are correctly positioned within the tobacco plug.

Advantageously, the upstream face of the disc of electrically insulating material also provides a large surface area on which the at least two heater electrical contacts can be provided, which facilitates the manufacture of the resistive heating element.

The disc of electrically insulating material may be formed from the same insulating material as the elongate electrically insulating substrate, or they may be formed from different materials. Preferably, the disc of electrically insulating material and the elongate electrically insulating substrate are formed from the same material. To simplify the manufacture of the resistive heating element, the disc of electrically insulating material and the elongate electrically insulating substrate are preferably integrally formed from a single piece of electrically insulating material. For example, the resistive heating element may formed by moulding or otherwise casting an electrically insulating material around the electrically resistive heating track.

In any of those embodiments in which the resistive heating element comprises a disc of electrically insulating material, the disc and the tobacco plug preferably both have a substantially circular cross-sectional shape, wherein the diameter of the disc is substantially the same as the diameter of the tobacco plug. This advantageously facilitates correct insertion of the resistive heating element into the tobacco plug. For example, a tubular guide may be used during manufacture of the aerosol-generating article to ensure that the disc of electrically insulating material is positioned coaxially with the tobacco plug.

The present invention also extends to methods of manufacturing tobacco plugs for use in manufacturing aerosol-generating articles comprising a resistive heater element within a tobacco plug, in accordance with any of the embodiments of the first aspect of the present invention described above and wherein the resistive heating element is incorporated into the tobacco plug as the tobacco plug is formed. Therefore, according to a fourth aspect of the present invention there is provided a method of manufacturing a tobacco plug for use in an aerosol-generating article according to the first aspect of the present invention, the method comprising positioning a resistive heating element on a forming belt, the resistive heating element comprising at least two heater electrical contacts for receiving a supply of electrical energy. A tobacco slurry is deposited onto the forming belt and dried to form a dried tobacco sheet comprising the resistive heating element. The dried tobacco sheet comprising the resistive heating element is formed into a tobacco plug, the resistive heating element positioned within the tobacco plug and comprising an upstream portion protruding from an upstream end of the tobacco plug, wherein the upstream portion of the resistive heating element comprises the least two heater electrical contacts for receiving a supply of electrical energy.

Forming the resistive heater element integrally with the tobacco plug can simplify the manufacture of the aerosol-generating article by reducing the number of assembly steps.

The resistive heating element may be positioned on the forming belt and the tobacco slurry then deposited onto the forming belt over the resistive heating element. Alternatively, the tobacco slurry may be deposited on the forming belt and the resistive heating element then positioned on the forming belt on top of the tobacco slurry.

The step of forming the dried tobacco sheet into a tobacco plug may comprise at least one of rolling and folding the dried tobacco sheet.

To accommodate the step of forming the dried tobacco sheet into a tobacco plug, the resistive heating element is preferably flexible. For example, the resistive heating element may comprise a flexible resistive wire.

The invention will now be further described, by way of example only, with reference to the accompanying drawings in which:
Figure 1 shows an aerosol-generating article in accordance with a first embodiment of the present invention;
Figure 2 shows a detailed view of the heater electrical contacts of the aerosol-generating article of Figure 1;
Figure 3 shows an alternative arrangement of heater electrical contacts for use with the aerosol-generating article of Figure 1;
Figure 4 shows an aerosol-generative device for use with the aerosol-generating article of Figure 1;
Figure 5 shows the aerosol-generating article of Figure 1 inserted into the aerosol-generating device of Figure 4 to form an electrically heated aerosol-generating system;
Figure 6 illustrates a method of forming the aerosol-generating article of Figure 1, in accordance with the present invention; and
Figure 7 shows an aerosol-generating article in accordance with a second embodiment of the present invention and configured for use with the aerosol-generating device of Figure 4.

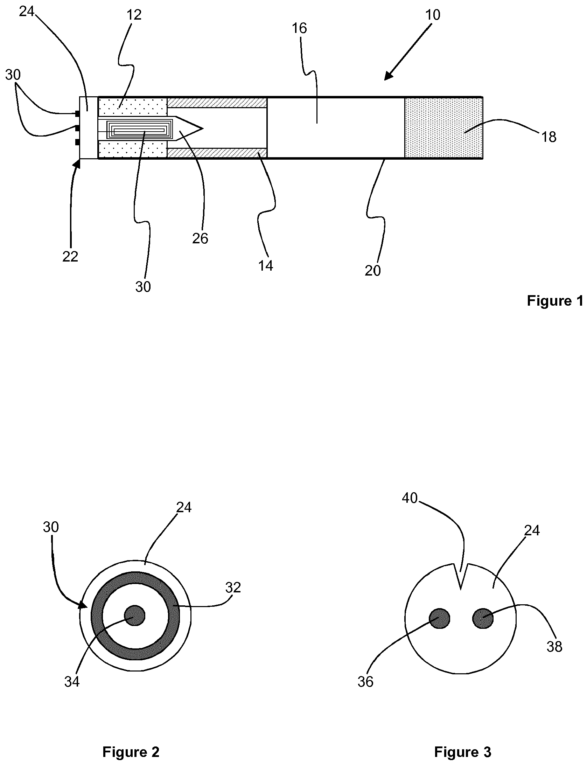

Figure 1 shows an aerosol-generating article 10 in accordance with a first embodiment of the present invention, the aerosol-generating article 10 comprising a tobacco plug 12, a hollow acetate tube 14, a polymeric filter 16, a mouthpiece 18 and an outer wrapper 20.

The aerosol-generating article 10 further comprises a resistive heating element 22 positioned within the tobacco plug 12, the resistive heating element comprising a disc of electrically insulating material 24 and an elongate electrically insulating substrate 26 extending from a downstream face of the disc of electrically insulating material 24. The disc of electrically insulating material 24 and the elongate electrically insulating substrate 26 are formed integrally as a single piece from a ceramic material.

An electrically resistive heating track 28 is embedded within the elongate electrically insulating substrate 26. Provided on an upstream face of the disc of electrically insulating material 24 are heater electrical contacts 30, which are connected to the electrically resistive heating track 28 for conducting a supply of electrical energy from an aerosol-generating device to the electrically resistive heating track 28.

Figure 2 shows a detailed view of the heater electrical contacts 30 on the upstream face of the disc of electrically insulating material 24. The heater electrical contacts 30 comprise a first heater electrical contact 32 having an annular shape and a second heater electrical contact 34 having a circular shape and positioned within the first heater electrical contact 32. The first and second heater electrical contacts 32, 34 are centred on the longitudinal axis of the disc of electrically insulating material 24 so that the orientation of the first and second heater electrical contacts 32, 34 is not dependent upon the rotational orientation of the aerosol-generating article 10 around its longitudinal axis.

Figure 3 shows an alternative arrangement of heater electrical contacts for the aerosol-generating article 10 of Figure 1. The alternative arrangement of heater electrical contacts comprises a first heater electrical contact 36 spaced apart from a second heater electrical contact 38. Depending upon the configuration of device electrical contacts in the aerosol-generating device with which the aerosol-generating article 10 is used, it may be necessary to insert the aerosol-generating article 10 into the aerosol-generating device with a particular rotational orientation when using the alternative heater electrical contact arrangement show in Figure 3. Therefore, in such embodiments the aerosol-generating article 10 can be provided with an indexing indicator to ensure correct rotational orientation of the aerosol-generating article 10. For example, as shown in Figure 3, a groove 40 may be provided in the disc of electrically insulating material 24, wherein the groove must be matched with a correspondingly shaped ridge on an aerosol-generating device to allow insertion of the aerosol-generating article 10 into the aerosol-generating device.

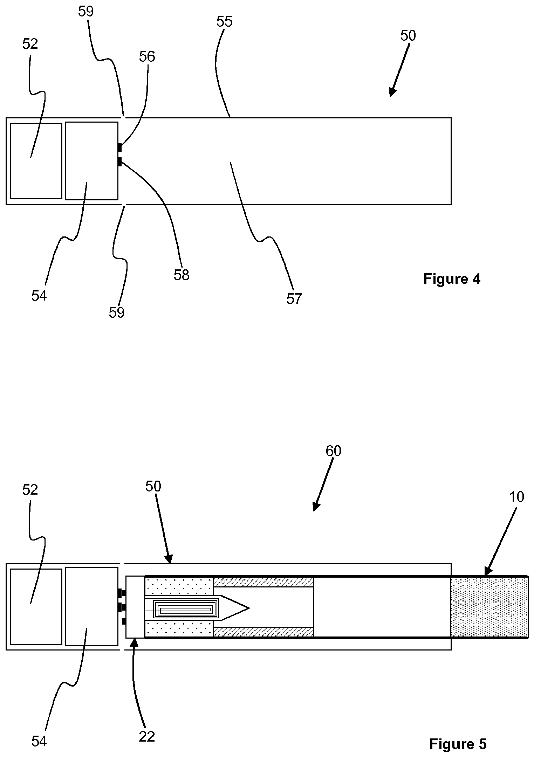

Figure 4 shows an aerosol-generating device 50 for use with the aerosol-generating article of Figure 1. The device 50 includes a supply of electrical energy 52 in the form of a rechargeable battery. A controller 54 controls the operation of the device 50, including the supply of electrical energy from the battery to the resistive heating element 22 of an aerosol-generating article 10. The aerosol-generating device 50 comprises a tubular housing 55 housing the supply of electrical energy 52 and the controller 54, the tubular housing 55 defining a cavity 57 for receiving the aerosol-generating article 10. A plurality of airflow inlets 59 are provided in the tubular housing 55 to allow air to flow into the cavity 57 during use of the aerosol-generating device 50 with the aerosol-generating article 10.

The aerosol-generating device 50 also comprises first and second device electrical contacts 56, 58 that are arranged to contact the heater electrical contacts 30 of the aerosol-generating article 10 when the article is inserted into the cavity 57 to form an electrically heated aerosol-generating system 60, as shown in Figure 5. During operation of the electrically heated aerosol-generating system 60, electrical energy is supplied from the battery to the resistive heating track 28 of the resistive heating element 22 via the first and second device electrical contacts 56, 58 and the first and second heater electrical contacts 32, 34.

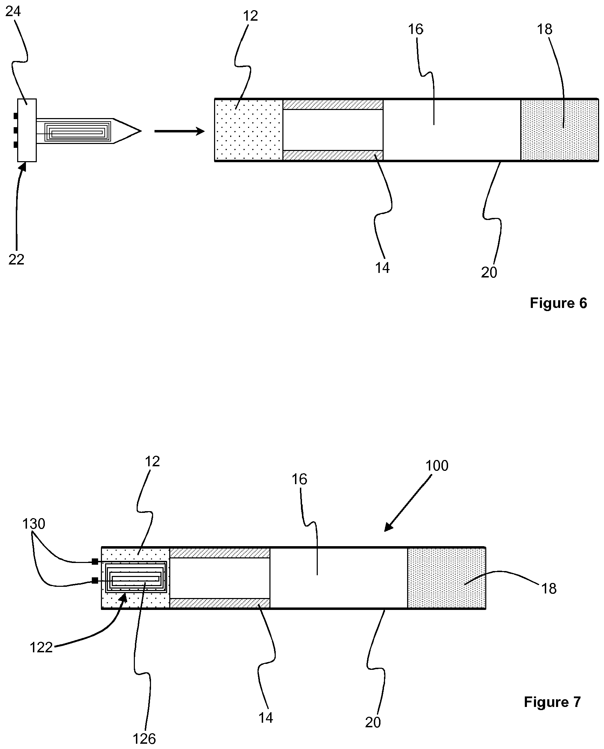

Figure 6 illustrates a method of forming the aerosol-generating article 10 of Figure 1 in which the tobacco plug 12, the hollow acetate tube 14, the polymeric filter 16, the mouthpiece 18 and the outer wrapper 20 are pre-assembled. The resistive heating element 22 is then inserted into the tobacco plug 12 until the downstream face of the disc of electrically insulating material 24 abuts the upstream face of the tobacco plug 12, thus forming the aerosol-generating article 10 of Figure 1.

Figure 7 illustrates an aerosol-generating article 100 in accordance with a second embodiment of the present invention. The aerosol-generating article 100 is substantially the same as the aerosol-generating article 10 shown in Figure 1, with the exception of the resistive heating element, and like reference numerals are used to designate like parts.

The aerosol-generating article 100 comprises a resistive heating element 122 comprising an electrically resistive wire 126 positioned within the tobacco plug 12. The electrically resistive wire 126 may be incorporated into the tobacco plug 12 during the process of forming the tobacco plug 12. For example, the electrically resistive wire 126 can be combined with a tobacco slurry on a forming belt, after which the tobacco slurry is dried to form a dried tobacco sheet comprising the electrically resistive wire 126, the dried tobacco sheet then being formed into the tobacco plug 12.

The resistive heating element 122 also comprises heater electrical contacts 130 provided at the upstream end of the aerosol-generating article 100 and connected to the electrically resistive wire 126. The heater electrical contacts 130 can be configured to permit use of the aerosol-generating article 100 with the aerosol-generating device 50 of Figure 4.

## Claims

1. An aerosol-generating article (10; 100) comprising:
a tobacco plug (12);
a mouthpiece (18) positioned downstream of the tobacco plug (12); and
a resistive heating element (22; 122) positioned within the tobacco plug (12), the resistive heating element (22; 122) comprising an upstream portion protruding from an upstream end of the tobacco plug (12), wherein the upstream portion of the resistive heating element (22; 122) comprises at least two heater electrical contacts (30; 130) for receiving a supply of electrical energy from at least two device electrical contacts (56, 58) when the aerosol-generating article (10; 100) comprising the resistive heating element (22; 122) is inserted into an aerosol-generating device (50).

2. An aerosol-generating article (10) according to claim 1, wherein the resistive heating element (22) comprises an electrically resistive heating track (28) provided on an elongate electrically insulating substrate (26), and wherein the electrically resistive heating track (28) is electrically connected to the at least two heater electrical contacts (30).

3. An aerosol-generating article (10) according to claim 2, wherein the elongate electrically insulating substrate (26) is shaped in the form of one of a rod, a needle, a pin, a blade, or a cone.

4. An aerosol-generating article (10) according to claim 2 or 3, wherein the upstream portion of the resistive heating element (22) comprises a disc of electrically insulating material (24), wherein the disc comprises a downstream face abutting an upstream end of the tobacco plug (12) and wherein the elongate electrically insulating substrate (26) extends downstream from the downstream face of the disc, and wherein the disc further comprises an upstream face on which the at least two heater electrical contacts (30) are provided.

5. An aerosol-generating article (10) according to claim 4, wherein the disc and the tobacco plug (12) both have a substantially circular cross-sectional shape, and wherein the diameter of the disc is substantially the same as the diameter of the tobacco plug (12).

6. An aerosol-generating article (10) according to any preceding claim, further comprising an article securing element configured to interact with a device securing element on an aerosol-generating device (50) to releasably retain the aerosol-generating article (10) within the aerosol-generating device (50).

7. An aerosol-generating article (10) according to any preceding claim, wherein at least one of the heater electrical contacts (30) and/or at least one of the device electrical contacts (56, 58) comprises a magnetised material configured to releasably retain the heater electrical contacts (30) in electrical contact with corresponding device electrical contacts (56, 58) when the aerosol-generating article (50) is inserted into the aerosol-generating device (50).

8. An aerosol-generating article (10) according to any preceding claim, wherein the aerosol-generating article (10) has a substantially circular cross-sectional shape centred on a longitudinal axis of the aerosol-generating article (10), wherein the at least two heater electrical contacts (30) comprise a first heater electrical contact (32) having an annular shape centred on the longitudinal axis of the aerosol-generating article (10) and a second heater electrical contact (34) having a circular or an annular shape centred on the longitudinal axis of the aerosol-generating article (10), wherein an outer diameter of the second heater electrical contact (34) is smaller than an inner diameter of the first heater electrical contact (32), and wherein the second heater electrical contact (34) is positioned within the first heater electrical contact (32).

9. An electrically heated aerosol-generating system (60) comprising:
an aerosol-generating article (10; 100) according to any of claims 1 to 7; and
an aerosol-generating device (50), the aerosol-generating device (50) comprising:
a tubular housing (55) comprising a cavity (57) for receiving at least an upstream portion of the aerosol-generating article (10; 100);
a supply of electrical energy (52) within the tubular housing (55); and
at least two device electrical contacts (56, 58) connected to the supply of electrical energy (52), wherein the at least two device electrical contacts (56, 58) are positioned at an upstream end of the cavity (57) and configured to contact the at least two heater electrical contacts (30; 130) when the aerosol-generating article (10; 100) is inserted into the cavity (57) to transfer electrical energy from the supply of electrical energy (52) to the resistive heating element (22; 122).

10. An electrically heated aerosol-generating system (60) according to claim 9, wherein the at least two device electrical contacts (56, 58) comprise a first device electrical contact (56) spaced apart from a second device electrical contact (58), wherein the aerosol-generating article (10) has a substantially circular cross-sectional shape centred on a longitudinal axis of the aerosol-generating article (10), wherein the at least two heater electrical contacts (30) comprise a first heater electrical contact (32) having an annular shape centred on the longitudinal axis of the aerosol-generating article (10) and a second heater electrical contact (34) having a circular or an annular shape centred on the longitudinal axis of the aerosol-generating article (10), wherein an outer diameter of the second heater electrical contact (34) is smaller than an inner diameter of the first heater electrical contact (32), and wherein the second heater electrical contact (34) is positioned within the first heater electrical contact (32).

11. A method of manufacturing an aerosol-generating article (10), the method comprising:
providing a tobacco plug (12) and a mouthpiece (18);
wrapping a wrapper (20) around at least a portion of the tobacco plug (12) and the mouthpiece (18) to secure the mouthpiece (18) to a downstream end of the tobacco plug (12);
providing a resistive heating element (22) comprising an electrically resistive heating track (28) on an elongate electrically insulating substrate (26), and at least two heater electrical contacts (30) for receiving a supply of electrical energy and connected to the electrically resistive heating track (28); and
inserting at least a portion of the elongate electrically insulating substrate (26) into the tobacco plug (12) at an upstream end of the tobacco plug (12), wherein an upstream portion of the resistive heating element (22) protrudes from the upstream end of the tobacco plug (12), and wherein the upstream portion of the resistive heating element (22) comprises the at least two heater electrical contacts (30) for receiving a supply of electrical energy.

12. A method according to claim 11, wherein the elongate electrically insulating substrate (26) is shaped in the form of one of a rod, a needle, a pin, a blade, or a cone, and wherein the step of inserting at least a portion of the elongate electrically insulating substrate (26) into the tobacco plug (12) comprises inserting the rod, needle, pin, blade or cone into the tobacco rod (12).

13. A method according to claim 11 or 12, wherein the upstream portion of the resistive heating element (22) comprises a disc of electrically insulating material (24), wherein the disc comprises an upstream face on which the at least two heater electrical contacts (30) are provided and a downstream face from which the elongate electrically insulating substrate (26) extends, and wherein the step of inserting at least a portion of the elongate electrically insulating substrate (26) into the tobacco plug (12) comprises inserting the elongate electrically insulating substrate (26) until the downstream face of the disc abuts the upstream end of the tobacco plug (12).

14. A method according to claim 13, wherein the disc and the tobacco plug (12) both have a substantially circular cross-sectional shape, and wherein the diameter of the disc is substantially the same as the diameter of the tobacco plug (12).

15. A method of manufacturing a tobacco plug (12) for use in an aerosol-generating article (10; 100) according to claim 1, the method comprising:
positioning a resistive heating element (22; 122) on a forming belt, the resistive heating element comprising at least two heater electrical contacts (30; 130) for receiving a supply of electrical energy;
depositing a tobacco slurry onto the forming belt;
drying the tobacco slurry to form a dried tobacco sheet comprising the resistive heating element (22; 122); and
forming the dried tobacco sheet comprising the resistive heating element (22; 122) into a tobacco plug (12), the resistive heating element (22; 122) positioned within the tobacco plug (12) and comprising an upstream portion protruding from an upstream end of the tobacco plug (12), wherein the upstream portion of the resistive heating element (22; 122) comprises the least two heater electrical contacts (30; 130) for receiving a supply of electrical energy.

## Patentansprüche

1. Aerosolerzeugungsartikel (10; 100), aufweisend:
einen Tabakeinsatz (12);
ein Mundstück (18), das nachgeschaltet des Tabakeinsatzes (12) positioniert ist; und
ein Widerstandsheizelement (22; 122), das innerhalb des Tabakeinsatzes (12) positioniert ist, wobei das Widerstandsheizelement (22; 122) einen zuströmseitigen Teil aufweist, der von einem zuströmseitigen Ende des Tabakeinsatzes (12) vorsteht, wobei der zuströmseitige Teil des Widerstandsheizelements (22; 122) mindestens zwei elektrische Heizvorrichtungskontakte (30; 130) zum Aufnehmen einer elektrischen Energieversorgung von mindestens zwei elektrischen Vorrichtungskontakten (56, 58) aufweist, wenn der aerosolerzeugende Artikel (10; 100), der das Widerstandsheizelement (22; 122) aufweist, in eine Aerosolerzeugungsvorrichtung (50) eingesetzt wird.

2. Aerosolerzeugender Artikel (10) nach Anspruch 1, wobei das Widerstandsheizelement (22) eine elektrische Widerstandsheizbahn (28) aufweist, die auf einem länglichen elektrisch isolierenden Substrat (26) vorgesehen ist, und wobei die elektrische Widerstandsheizbahn (28) mit den mindestens zwei elektrischen Heizvorrichtungskontakten (30) elektrisch verbunden ist.

3. Aerosolerzeugender Artikel (10) nach Anspruch 2, wobei das längliche elektrisch isolierende Substrat (26) in der Form von einem von einem Stock, einer Nadel, einem Stift, einem Schwert oder einem Konus geformt ist.

4. Aerosolerzeugender Artikel (10) nach Anspruch 2 oder 3, wobei der zuströmseitige Teil des Widerstandsheizelements (22) eine Scheibe aus elektrisch isolierendem Material (24) aufweist, wobei die Scheibe eine nachgeschaltete Seite aufweist, die an einem zuströmseitigen Ende des Tabakeinsatzes (12) anliegt, und wobei sich das längliche elektrisch isolierende Substrat (26) nachgeschaltet der nachgeschalteten Seite der Scheibe erstreckt, und wobei die Scheibe weiter eine zuströmseitige Seite aufweist, auf der die mindestens zwei elektrischen Heizvorrichtungskontakte (30) vorgesehen sind.

5. Aerosolerzeugender Artikel (10) nach Anspruch 4, wobei sowohl die Scheibe als auch der Tabakeinsatz (12) eine im Wesentlichen kreisförmige Querschnittsform aufweisen, wobei der Durchmesser der Scheibe im Wesentlichen der gleiche ist wie der Durchmesser des Tabakeinsatzes (12).

6. Aerosolerzeugender Artikel (10) nach einem der vorstehenden Ansprüche, weiter aufweisend ein Artikelbefestigungselement, das ausgelegt ist, mit einem Vorrichtungsbefestigungselement an einer Aerosolerzeugungsvorrichtung (50) zu interagieren, um den aerosolerzeugenden Artikel (10) innerhalb der Aerosolerzeugungsvorrichtung (50) lösbar zu halten.

7. Aerosolerzeugender Artikel (10) nach einem der vorstehenden Ansprüche, wobei mindestens einer von den elektrischen Heizvorrichtungskontakten (30) und/oder mindestens einer von den elektrischen Vorrichtungskontakten (56, 58) ein magnetisiertes Material aufweist, das ausgelegt ist, die elektrischen Heizvorrichtungskontakte (30) in elektrischem Kontakt mit entsprechenden elektrischen Vorrichtungskontakten lösbar zu halten (56, 58), wenn der aerosolerzeugende Artikel (50) in die Aerosolerzeugungsvorrichtung (50) eingesetzt wird.

8. Aerosolerzeugender Artikel (10) nach einem der vorstehenden Ansprüche, wobei der aerosolerzeugende Artikel (10) eine auf einer Längsachse des aerosolerzeugenden Artikels (10) zentrierte im Wesentlichen kreisförmige Querschnittsform aufweist, wobei die mindestens zwei elektrischen Heizvorrichtungskontakte (30) einen ersten elektrischen Heizvorrichtungskontakt (32) mit einer ringförmigen Form aufweisen, die auf der Längsachse des aerosolerzeugenden Artikels (10) zentriert ist, und einen zweiten elektrischen Heizvorrichtungskontakt (34) mit einer kreisförmigen oder einer ringförmigen Form, die auf der Längsachse des aerosolerzeugenden Artikels (10) zentriert ist, wobei ein Außendurchmesser des zweiten elektrischen Heizvorrichtungskontakts (34) kleiner ist als ein Innendurchmesser des ersten elektrischen Heizvorrichtungskontakts (32), und wobei der zweite elektrische Heizvorrichtungskontakt (34) innerhalb des ersten elektrischen Heizvorrichtungskontakts (32) positioniert ist.

9. Elektrisch beheiztes Aerosolerzeugungssystem (60); aufweisend:
einen aerosolerzeugenden Artikel (10; 100) nach einem der Ansprüche 1 bis 7; und
eine Aerosolerzeugungsvorrichtung (50), wobei die Aerosolerzeugungsvorrichtung (50); aufweist:
ein röhrenförmiges Gehäuse (55), das einen Hohlraum (57) zum Aufnehmen mindestens eines zuströmseitigen Teils des aerosolerzeugenden Artikels (10; 100) aufweist;
eine elektrische Energieversorgung (52) innerhalb des röhrenförmigen Gehäuses (55); und
mindestens zwei elektrische Vorrichtungskontakte (56, 58), die mit der elektrischen Energieversorgung (52) verbunden sind, wobei die mindestens zwei elektrischen Vorrichtungskontakte (56, 58) an einem zuströmseitigen Ende des Hohlraums (57) positioniert und ausgelegt sind, die mindestens zwei elektrischen Heizvorrichtungskontakte zu kontaktieren (30; 130), wenn der aerosolerzeugende Artikel (10; 100) in den Hohlraum (57) eingesetzt wird, um elektrische Energie von der elektrischen Energieversorgung (52) zum Widerstandsheizelement (22; 122) zu übertragen.

10. Elektrisch beheiztes Aerosolerzeugungssystem (60) nach Anspruch 9, wobei die mindestens zwei elektrischen Vorrichtungskontakte (56, 58) einen ersten elektrischen Vorrichtungskontakt (56) aufweisen, der von einem zweiten elektrischen Vorrichtungskontakt (58) beabstandet ist, wobei der aerosolerzeugende Artikel (10) eine auf einer Längsachse des aerosolerzeugenden Artikels (10) zentrierte im Wesentlichen kreisförmige Querschnittsform aufweist, wobei die mindestens zwei elektrischen Heizvorrichtungskontakte (30) einen ersten elektrischen Heizvorrichtungskontakt (32) mit einer ringförmigen Form aufweisen, die auf der Längsachse des aerosolerzeugenden Artikels (10) zentriert ist, und einen zweiten elektrischen Heizvorrichtungskontakt (34) mit einer kreisförmigen oder einer ringförmigen Form, die auf der Längsachse des aerosolerzeugenden Artikels (10) zentriert ist, wobei ein Außendurchmesser des zweiten elektrischen Heizvorrichtungskontakts (34) kleiner ist als ein Innendurchmesser des ersten elektrischen Heizvorrichtungskontakts (32), und wobei der zweite elektrische Heizvorrichtungskontakt (34) innerhalb des ersten elektrischen Heizvorrichtungskontakts (32) positioniert ist.

11. Verfahren zum Herstellen eines aerosolerzeugenden Artikels (10), wobei das Verfahren aufweist:
Bereitstellen eines Tabakeinsatzes (12) und eines Mundstücks (18);
Hüllen einer Umhüllung (20) um mindestens einen Abschnitt des Tabakeinsatzes (12) und des Mundstücks (18), um das Mundstück (18) an einem nachgeschalteten Ende des Tabakeinsatzes (12) zu befestigen;
Bereitstellen eines Widerstandsheizelements (22), das eine elektrische Widerstandsheizbahn (28) auf einem länglichen elektrisch isolierenden Substrat (26) und mindestens zwei elektrische Heizvorrichtungskontakte (30) zum Aufnehmen einer elektrischen Energieversorgung, die mit der elektrischen Widerstandsheizbahn (28) verbunden sind, aufweist; und
Einsetzen mindestens eines Abschnitts des länglichen elektrisch isolierenden Substrats (26) in den Tabakeinsatz (12) an einem zuströmseitigen Ende des Tabakeinsatzes (12), wobei ein zuströmseitiger Teil des Widerstandsheizelements (22) von dem zuströmseitigen Ende des Tabakeinsatzes (12) vorsteht, und wobei der zuströmseitige Teil des Widerstandsheizelements (22) die mindestens zwei elektrischen Heizvorrichtungskontakte (30) zum Aufnehmen einer elektrischen Energieversorgung aufweist.

12. Verfahren nach Anspruch 11, wobei das längliche elektrisch isolierende Substrat (26) in der Form von einem von einem Stock, einer Nadel, einem Stift, einem Schwert oder einem Konus geformt ist, und wobei der Schritt des Einsetzens von mindestens einem Abschnitt des länglichen elektrisch isolierenden Substrats (26) in den Tabakeinsatz (12) das Einsetzen des Stocks, der Nadel, des Stifts, des Schwerts oder des Konus in den Tabakstock (12) aufweist.

13. Verfahren nach Anspruch 11 oder 12, wobei der zuströmseitige Teil des Widerstandsheizelements (22) eine Scheibe aus elektrisch isolierendem Material (24) aufweist, wobei die Scheibe eine zuströmseitige Seite aufweist, auf der die mindestens zwei elektrischen Heizvorrichtungskontakte (30) vorgesehen sind, und eine nachgeschaltete Seite, von der sich das längliche elektrisch isolierende Substrat (26) erstreckt, und wobei der Schritt des Einsetzens von mindestens einem Abschnitt des länglichen elektrisch isolierenden Substrats (26) in den Tabakeinsatz (12) das Einsetzen des länglichen elektrisch isolierenden Substrats (26), bis die nachgeschaltete Seite der Scheibe an dem zuströmseitigen Ende des Tabakeinsatzes (12) anliegt, aufweist.

14. Verfahren nach Anspruch 13, wobei sowohl die Scheibe als auch der Tabakeinsatz (12) eine im Wesentlichen kreisförmige Querschnittsform aufweisen, wobei der Durchmesser der Scheibe im Wesentlichen der gleiche ist wie der Durchmesser des Tabakeinsatzes (12) .

15. Verfahren zum Herstellen eines Tabakeinsatzes (12) zum Gebrauch in einem aerosolerzeugenden Artikel (10; 100) nach Anspruch 1, wobei das Verfahren aufweist:
Positionieren eines Widerstandsheizelements (22; 122) auf einem Formband, wobei das Widerstandsheizelement mindestens zwei elektrische Heizvorrichtungskontakte (30; 130) zum Aufnehmen einer elektrischen Energieversorgung aufweist;
Aufbringen eines Tabakschlamms auf das Formband;
Trocknen des Tabakschlamms, um ein getrocknetes Tabakflächengebilde zu bilden, welches das Widerstandsheizelement (22; 122) aufweist; und
Formen des getrockneten Tabakflächengebildes, welches das Widerstandsheizelement (22; 122) aufweist, in einen Tabakeinsatz (12), wobei das Widerstandsheizelement (22; 122) innerhalb des Tabakeinsatzes (12) positioniert wird und einen zuströmseitigen Teil aufweist, der von einem zuströmseitigen Ende des Tabakeinsatzes (12) vorsteht, wobei der zuströmseitige Teil des Widerstandsheizelements (22; 122) die mindestens zwei elektrischen Heizvorrichtungskontakte (30; 130) zum Aufnehmen einer elektrischen Energieversorgung aufweist.

## Revendications

1. Article de génération d'aérosol (10 ; 100) comprenant :
un bouchon en tabac (12) ;
un embout buccal (18) positionné en aval du bouchon en tabac (12) ; et
un élément chauffant résistif (22 ; 122) positionné à l'intérieur du bouchon en tabac (12), l'élément chauffant résistif (22 ; 122) comprenant une partie en amont dépassant d'une extrémité en amont du bouchon en tabac (12), dans lequel la partie en amont de l'élément chauffant résistif (22 ; 122) comprend au moins deux contacts électriques d'éléments chauffants (30 ; 130) pour la réception d'une alimentation d'énergie électrique d'au moins deux contacts électriques de dispositif (56 , 58) lorsque l'article de génération d'aérosol (10 ; 100) comprenant l'élément chauffant résistif (22 ; 122) est inséré dans un dispositif de génération d'aérosol (50).

2. Article de génération d'aérosol (10) selon la revendication 1, dans lequel l'élément chauffant résistif (22) comprend une piste chauffante électriquement résistive (28) fournie sur un substrat électriquement isolant allongé (26), et dans lequel la piste chauffante électriquement résistive (28) est électriquement connectée à l'au moins deux contacts électriques d'éléments chauffants (30).

3. Article de génération d'aérosol (10) selon la revendication 2, dans lequel le substrat électriquement isolant allongé (26) se présente sous la forme d'un parmi une tige, une aiguille, une broche, une lame ou un cône.

4. Article de génération d'aérosol (10) selon la revendication 2 ou 3, dans lequel la partie en amont de l'élément chauffant résistif (22) comprend un disque de matériau électriquement isolant (24), dans lequel le disque comprend une face en aval venant en butée contre une extrémité en amont du bouchon en tabac (12) et dans lequel le substrat électriquement isolant allongé (26) s'étend en aval de la face en aval du disque, et dans lequel le disque comprend en outre une face en amont sur laquelle l'au moins deux contacts électriques d'éléments chauffants (30) sont fournis.

5. Article de génération d'aérosol (10) selon la revendication 4, dans lequel le disque et le bouchon en tabac (12) ont tous les deux une forme de section transversale sensiblement circulaire, et dans lequel le diamètre du disque est substantiellement identique au diamètre du bouchon en tabac (12).

6. Article de génération d'aérosol (10) selon l'une quelconque revendication précédente, comprenant en outre un élément de fixation d'article configuré pour interagir avec un élément de fixation de dispositif sur un dispositif de génération d'aérosol (50) pour retenir de manière libérable l'article de génération d'aérosol (10) à l'intérieur du dispositif de génération d'aérosol (50).

7. Article de génération d'aérosol (10) selon l'une quelconque des revendications précédentes, dans lequel au moins un parmi les contacts électriques d'éléments chauffants (30) et/ou au moins un parmi les contacts électriques de dispositif (56 , 58) comprend une matière magnétisée configurée pour retenir de manière libérable les contacts électriques d'éléments chauffants (30) en contact électrique avec des contacts électriques correspondants du dispositif (56 , 58) lorsque l'article de génération d'aérosol (50) est inséré dans le dispositif de génération d'aérosol (50).

8. Article de génération d'aérosol (10) selon l'une quelconque des revendications précédentes, dans lequel l'article de génération d'aérosol (10) présente une forme de section transversale sensiblement circulaire centrée sur un axe longitudinal de l'article de génération d'aérosol (10), dans lequel l'au moins deux contacts électriques d'éléments chauffants (30) comprennent un premier contact électrique d'un élément chauffant (32) ayant une forme annulaire centrée sur l'axe longitudinal de l'article de génération d'aérosol (10) et un deuxième contact électrique d'un élément chauffant (34) ayant une forme circulaire ou annulaire centrée sur l'axe longitudinal de l'article de génération d'aérosol (10), dans lequel un diamètre extérieur du deuxième contact électrique d'un élément chauffant (34) est inférieur à un diamètre intérieur du premier contact électrique d'un élément chauffant (32), et dans lequel le deuxième contact électrique d'un élément chauffant (34) est positionné à l'intérieur du premier contact électrique d'un élément chauffant (32) .

9. Système de génération d'aérosol chauffé électriquement (60) comprenant :
un article de génération d'aérosol (10 ; 100) selon l'une quelconque des revendications 1 à 7 ; et
un dispositif de génération d'aérosol (50), le dispositif de génération d'aérosol (50) comprenant :
un boîtier tubulaire (55) comprenant une cavité (57) pour la réception d'au moins une partie en amont de l'article de génération d'aérosol (10 ; 100) ;
une alimentation d'énergie électrique (52) à l'intérieur du boîtier tubulaire (55) ; et
au moins deux contacts électriques de dispositif (56 , 58) connecté à l'alimentation d'énergie électrique (52), où l'au moins deux contacts électriques de dispositif (56 , 58) sont positionnés à une extrémité en amont de la cavité (57) et configurés pour être en contact avec l'au moins deux contacts électriques d'éléments chauffants (30 ; 130) lorsque l'article de génération d'aérosol (10 ; 100) est inséré dans la cavité (57) afin de transmettre l'énergie électrique de l'alimentation d'énergie électrique (52) à l'élément chauffant résistif (22 ; 122).

10. Système de génération d'aérosol chauffé électriquement (60) selon la revendication 9, dans lequel l'au moins deux contacts électriques de dispositif (56, 58) comprennent un premier contact électrique du dispositif (56) espacé d'un deuxième contact électrique du dispositif (58), dans lequel l'article de génération d'aérosol (10) présente une forme de section transversale sensiblement circulaire centrée sur un axe longitudinal de l'article de génération d'aérosol (10), dans lequel l'au moins deux contacts électriques d'éléments chauffants (30) comprennent un premier contact électrique d'un élément chauffant (32) ayant une forme annulaire centrée sur l'axe longitudinal de l'article de génération d'aérosol (10) et un deuxième contact électrique d'un élément chauffant (34) ayant une forme circulaire ou annulaire centrée sur l'axe longitudinal de l'article de génération d'aérosol (10), dans lequel un diamètre extérieur du deuxième contact électrique d'un élément chauffant (34) est inférieur à un diamètre intérieur du premier contact électrique d'un élément chauffant (32), et dans lequel le deuxième contact électrique d'un élément chauffant (34) est positionné à l'intérieur du premier contact électrique d'un élément chauffant (32).

11. Procédé de fabrication d'un article de génération d'aérosol (10), le procédé comprenant :
la fourniture d'un bouchon en tabac (12) et d'un embout buccal (18) ;
l'emballage d'une enveloppe (20) autour d'au moins une partie du bouchon en tabac (12) et de l'embout buccal (18) pour fixer l'embout buccal (18) à une extrémité en aval du bouchon en tabac (12) ;
la fourniture d'un élément chauffant résistif (22) comprenant une piste chauffante électriquement résistive (28) sur un substrat électriquement isolant allongé (26), et au moins deux contacts électriques d'éléments chauffants (30) pour la réception d'une alimentation d'énergie électrique et connectés à la piste chauffante électriquement résistive (28) ; et
l'insertion d'au moins une partie du substrat électriquement isolant allongé (26) dans le bouchon en tabac (12) à une extrémité en amont du bouchon en tabac (12), dans lequel une partie en amont de l'élément chauffant résistif (22) dépasse de l'extrémité en amont du bouchon en tabac (12), et dans lequel la partie en amont de l'élément chauffant résistif (22) comprend l'au moins deux contacts électriques d'éléments chauffants (30) pour la réception d'une alimentation d'énergie électrique.

12. Procédé selon la revendication 11, dans lequel le substrat électriquement isolant allongé (26) se présente sous la forme d'un parmi une tige, une aiguille, une broche, une lame ou un cône, et dans lequel l'étape d'insertion d'au moins une partie du substrat électriquement isolant allongé (26) dans le bouchon en tabac (12) comprend l'insertion de la tige, l'aiguille, la broche, la lame ou du cône dans la tige de tabac (12).

13. Procédé selon la revendication 11 ou 12, dans lequel la partie en amont de l'élément chauffant résistif (22) comprend un disque de matériau électriquement isolant (24), dans lequel le disque comprend une face en amont sur laquelle l'au moins deux contacts électriques d'éléments chauffants (30) sont fournis et une face en aval depuis laquelle s'étend le substrat électriquement isolant allongé (26), et dans lequel l'étape de l'insertion d'au moins une partie du substrat électriquement isolant allongé (26) dans le bouchon en tabac (12) comprend l'insertion du substrat électriquement isolant allongé (26) jusqu'à ce que la face en aval du disque vienne en butée contre l'extrémité en amont du bouchon en tabac (12).

14. Procédé selon la revendication 13, dans lequel le disque et le bouchon en tabac (12) ont tous les deux une forme de section transversale sensiblement circulaire, et dans lequel le diamètre du disque est substantiellement identique au diamètre du bouchon en tabac (12).

15. Procédé pour la fabrication d'un bouchon en tabac (12) destiné à être utilisé dans un article de génération d'aérosol (10 ; 100) selon la revendication 1, le procédé comprenant :
le positionnement d'un élément chauffant résistif (22 ; 122) sur une courroie de formation, l'élément chauffant résistif comprenant au moins deux contacts électriques d'éléments chauffants (30 ; 130) pour la réception d'une alimentation d'énergie électrique ;
le dépôt d'une boue de tabac sur la courroie de formation ;
le séchage de la boue de tabac pour former une feuille de tabac séché comprenant l'élément chauffant résistif (22 ; 122) ; et
la formation de la feuille de tabac séché comprenant l'élément chauffant résistif (22 ; 122) dans un bouchon en tabac (12), l'élément chauffant résistif (22; 122) positionné à l'intérieur du bouchon en tabac (12) et comprenant une partie en amont dépassant d'une extrémité en amont du bouchon en tabac (12), dans lequel la partie en amont de l'élément chauffant résistif (22 ; 122) comprend l'au moins deux contacts électriques d'éléments chauffants (30 ; 130) pour la réception d'une alimentation d'énergie électrique.
